# EUROPÄISCHE PATENTSCHRIFT

(11) **EP 1 289 477 B1**
(45) Veröffentlichungstag und Bekanntmachung des Hinweises auf die Patenterteilung: **25.06.2008**
(21) Anmeldenummer: 01960276.2
(22) Anmeldetag: 07.06.2001
(51) Int. Cl.: A61K 6/033, A61K 8/24, A61K 8/88, A61K 33/16, A61K 33/42, A61Q 11/00

(54) **ZUSAMMENSETZUNGEN ZUR BEHANDLUNG VON ZAHN- UND/ODER KNOCHENGEWEBE**
COMPOSITIONS FOR TREATING TOOTH AND/OR BONE TISSUE
COMPOSITIONS POUR LE TRAITEMENT DE TISSUS DENTAIRES ET/OU OSSEUX

(30) Priorität: 16.06.2000 DE 10028975
(43) Veröffentlichungstag der Anmeldung: 12.03.2003
(73) Patentinhaber: Henkel AG & Co. KGaA, 40589 Düsseldorf (DE)
(72) Erfinder: ROTH, Marcel, 40589 Düsseldorf (DE); KROPF, Christian, 40721 Hilden (DE); WÜLKNITZ, Peter, 42799 Leichlingen (DE); KINTRUP, Lothar, 40595 Düsseldorf (DE); DOLHAINE, Hans, 41352 Korschenbroich (DE); SCHWARK, Hans-Jürgen, 40789 Monheim (DE); PASTURA, Amerigo, 58453 Witten (DE); MEINDERS, Michael, 47800 Krefeld (DE); LASKA, Hans, 40627 Düsseldorf (DE); MÜLLNER, Stefan, 40764 Langenfeld (DE)
(86) Internationale Anmeldenummer: PCT/EP2001/006424
(87) Internationale Veröffentlichungsnummer: WO 2001/095863

(56) Entgegenhaltungen:
- EP-A- 1 002 513
- EP-A- 1 086 711
- WO-A-00/03747
- WO-A-00/37033
- WO-A-00/46147
- WO-A-01/01930
- GARCIA CARRODEGUAS, R. ET AL.: "Effect of polymeric additions on the properties of calcium salt sements" REVISTA CENIC CIENCIAS QUIMICAS, Bd. 31, Nr. 1, 2000, XP001041820

## Beschreibung

Die Erfindung betrifft Zusammensetzungen, welche in Wasser schwerlösliche Calciumsalze sowie Polyelektrolyte enthalten und sich zur Glättung, Wiederherstellung und remineralisierenden Behandlung von Zahn- und Knochengewebe eignen.

Der Zahnschmelz sowie das Stützgewebe der Knochen bestehen überwiegend aus dem Mineral Hydroxylapatit. Im biologischen Entstehungsprozeß lagert sich Hydroxylapatit in geordneter Weise an die Proteinmatrix im Knochen oder Zahn an, die überwiegend aus Kollagen besteht. Die Ausbildung der harten und belastungsfähigen mineralischen Strukturen wird dabei durch die sogenannten Matrixproteine gesteuert, welche neben Kollagen durch weitere Proteine gebildet werden, die sich an das Kollagen anlagern und so einen strukturierten Mineralisierungsprozeß, der auch als Biomineralisation bezeichnet wird, bewirken.

In der Mund- und Zahnpflege werden Phosphatsalze des Calciums seit langem zur Förderung der Remineralisierung des Zahnschmelzes den Rezepturen von Zahnreinigungsmitteln und Zahnpflegemitteln zugesetzt. Wegen der kurzen Einwirkzeit dieser Rezepturen und der Langsamkeit des Biomineralisationsprozesses muß jedoch die Anwendung der Calciumsalze häufig wiederholt werden, und es lassen sich häufig nur unbefriedigende Remineralisationseffekte erreichen.

Bei der Behandlung oder Wiederherstellung von Zahn- und Knochenmaterial spielen sogenannte Knochenersatzmittel, welche den natürlichen Biomineralisationsprozeß induzieren bzw. fördern, eine wichtige Rolle. Hydroxylapatit wurde zur Herstellung unterschiedlicher Formen von Implantaten benutzt. Bei der in der Literatur beschriebenen Herstellung durch Plasma-Sprühverfahren wird das Hydroxylapatit-Pulver jedoch hohen Temperaturen ausgesetzt, bei welchen sich der Hydroxylapatit unter Bildung von Tricalciumphosphat zersetzt (S. Zhang und K. E. Gonsalves, J. Mater. Sci. Mater. Med. 8 (1997), 25). Dem natürlichen Zahn- oder Knochengewebe ähnliche Ersatzstoffe auf der Grundlage von Apatit, welche sich bei Temperaturen nahe der Raumtemperatur herstellen und verarbeiten lassen und beispielsweise unter den Arbeitsbedingungen eines Dentallabors oder einer Zahnarztpraxis anwendbar sind, sind aus dem Stand der Technik bisher nicht bekannt.

Herkömmliche Mittel zur Restaurierung von Zahnmaterial bestehen im wesentlichen aus synthetischen Monomeren, die nach Applikation am Zahn zur Polymerisation gebracht werden. So beschreibt beispielsweise die WO 99/17716 Dentalmaterialien, bei welchen nanopartikuläre Füllstoffe wie z. B. Glas, Zeolith oder Metalloxide in eine Polymermatrix eingebettet werden und dem durch Polymerisation ausgehärteten Dentalmaterial eine erhöhte Abriebbeständigkeit und Festigkeit verleihen.

Die auf diese und ähnliche Weise erhaltenen Polymere besteht aus nicht natürlichen, vom Zahnmaterial grundlegend verschiedenen Stoffen, welche durch den natürlichen Biomineralisationsprozeß nicht innig mit dem natürlichen Zahnmaterial verbunden werden können. Darüber hinaus stellen Restmonomere ein unerwünschtes toxikologisches Risiko dar.

Es wurde gefunden, daß bestimmte Zusammensetzungen mit einem Gehalt an Calciumsalzen sowie Polyelektrolyten zur Überwindung von vorstehend genannten Nachteilen des Stands der Technik geeignet sind.

In WO 00/03747 werden Zusammensetzungen beschrieben, die neben Apatitbasierten nanostrukturierten Materialien auch bestimmte Polyelektrolyte enthalten können. Hier erfolgt die Herstellung der Materialien jedoch durch Zerkleinerung von makrokristallinen Strukturen und die Polyelektrolyte sind nicht während der Herstellung der nanostrukturierten Materialien anwesend.

García Carrodeguas et al. beschreiben in Revista CENIC Ciencias Quimicas 31 (1) (2000), 57 - 62 ein Verfahren zur Beschichtung von Calciumphosphat-Partikeln im oberen Mikrometerbereich, bei dem bereits ausgebildete Calciumphosphat-Partikel mit einem Polyelektrolyten vermischt werden.

In WO 00/37033 wird ein Verfahren beschrieben, bei dem die Fällung von Calciumphosphat in Gegenwart von wasserlöslichen Tensiden oder wasserlöslichen polymeren Schutzkolloiden durchgeführt wird. Als wasserlösliche Schutzkolloide werden hierbei auch Polyacrylsäure, Carboxymethylcellulose und Polyasparaginsäure genannt. In WO 00/37033 wird jedoch nicht beschrieben, dass Einengung erfolgt, bis eine pasten- oder gelartige Konsistenz erreicht wird.

Auch in WO 01/01930 sind als Oberflächenmodifikationsmittel nur Polyasparaginsäure, Carboxymethylcellulose und Polyacrylate genannt. Ebenso erfolgt in WO 01/01930 keine Einengung, bis eine pasten- oder gelartige Konsistenz erreicht wird.

In WO 00/46147 sind kugelförmige Calciumphosphat-Partikel genannt, jedoch keine stäbchenförmige.

Gegenstand der vorliegenden Erfindung ist daher eine Zusammensetzung zur Behandlung von Zahn- und/oder Knochengewebe, welche
(a) ein in Wasser schwerlösliches Calciumsalz, ausgewählt aus Phosphaten, Fluoriden und Fluorophosphaten, die wahlweise zusätzlich Hydroxyl- und/oder Carbonat-Gruppen enthalten können, wobei die Calciumsalze in Form von nanopartikulären, stäbchenförmigen Primärteilchen mit einem mittleren Teilchendurchmesser im Bereich von 5 bis 300 nm vorliegen, und
(b) einen Polyelektrolyten ausgewählt aus Alginsäuren, Pektinen, Carrageenan, Polygalakturonsäuren, Amino- und Aminosäurederivaten von Alginsäuren, Pektinen, Carrageenan und Polygalakturonsäuren, Polyaspartamiden, Nucleinsäuren, Ligninsulfonaten, Amino- und/oder Carboxylgruppen-haltigen Cyclodextrin- oder Dextran-Derivaten, Polymethacrylsäuren, Polymaleinaten, Polyvinylsulfonsäuren, Polyvinylphosphonsäuren, Polyethyleniminen, Polyvinylaminen,
   umfasst, wobei die Zusammensetzung erhältlich ist durch ein Verfahren, bestehend aus den Schritten
   - Fällung einer wässrigen Lösung eines Calciumsalzes in Gegenwart eines Polyelektrolyten
   - Verminderung des Wassergehalts bis zum Erreichen einer pasten- oder gelartigen Konsistenz.

Im Sinne der Erfindung versteht sich, daß die Zusammensetzung auch mehrere Calciumsalze und/oder mehrere Polyelektrolyten umfassen kann.

Als in Wasser schwerlöslich sollen solche Salze verstanden werden, die bei 20° C zu weniger als 1 g/l und insbesondere zu weniger als 1 mg/l löslich sind.

Bevorzugt geeignete Calciumsalze sind Apatit, Calciumhydroxyphosphat (Ca₅[OH(PO₄)₃]) bzw. Hydroxylapatit, Calciumfluorphosphat (Ca₅[F(PO₄)₃]) bzw. Fluorapatit, Fluor-dotierter Hydroxylapatit der allgemeinen Zusammensetzung Ca₅(PO₄)₃(OH,F) und Calciumfluorid (Ca F₂) bzw. Fluorit (Flußspat).

Unter Primärteilchen werden die Kristallite, d. h. die Einzelkristalle der genannten Calciumsalze verstanden. Als Teilchendurchmesser soll hier der Durchmesser der Teilchen in Richtung ihrer größten Längenausdehnung verstanden werden. Unter dem mittleren Teilchendurchmesser ist ein über die Gesamtmenge des Calciumsalzes gemittelter Wert zu verstehen. Die Bestimmung der Teilchendurchmesser kann durch dem Fachmann geläufige Methoden erfolgen, beispielsweise durch die Methode der dynamischen Lichtstreuung.

Vorzugsweise liegt der mittlere Teilchendurchmesser der nanopartikulären Primärteilchen im Bereich von 10 bis 150 nm, und besonders bevorzugt liegen sie als stäbchenförmige Partikel vor mit einer Dicke im Bereich von 5 bis 50 nm und einer Länge im Bereich von 10 bis 150 nm. Unter Dicke ist hier der kleinste Durchmesser der Stäbchen zu verstehen, unter Länge ihr größter Durchmesser.

Bevorzugt werden im Rahmen der vorliegenden Erfindung Polyelektrolyte eingesetzt, die zur Salzbildung mit zweiwertigen Kationen geeignete Gruppen tragen. Insbesondere eignen sich Carboxylat-Gruppen tragende Polymere.

Im Sinne der Erfindung besonders bevorzugte Polyelektrolyte sind Alginsäuren, Pektine, Desoxyribonukleinsäuren, Ribonukleinsäuren und Polymethacrylsäuren.

Der in den erfindungsgemäßen Zusammensetzungen enthaltene Polyelektrolyt kann teilweise als Umhüllung der Calciumsalz-Nanopartikel vorliegen, teilweise aber auch unabhängig von den Nanopartikeln frei in der Zusammensetzung vorliegen.

In der Regel werden die Calciumsalze in einer Konzentration von insgesamt 1 bis 40, vorzugsweise jedoch 5 bis 25 Gew.-%, bezogen auf das Gesamtgewicht der Zusammensetzung, eingesetzt.

Die Polyelektrolyte werden in der Regel in einer Konzentration von insgesamt 0,1 bis 40, vorzugsweise jedoch 5 bis 20 Gew.-%, bezogen auf das Gesamtgewicht der Zusammensetzung, eingesetzt.

Die Zusammensetzungen enthalten bevorzugt 2 bis 50 Gew.-% und besonders bevorzugt 5 bis 10 Gew.-% Polyelektrolyt bezogen auf das Gesamtgewicht aus Calciumsalz und Polyelektrolyt. Es versteht sich im Sinne der Erfindung, daß an die Stelle eines Polyelektrolyten auch ein Gemisch unterschiedlicher Polyelektrolyten treten kann.

Die erfindungsgemäßen Zusammensetzungen enthalten in der Regel zwischen 10 und 95 Gew.-%, vorzugsweise zwischen 50 und 80 Gew.-%, Wasser, bezogen auf das Gesamtgewicht der Zusammensetzung.

Besonders bevorzugt liegt die Zusammensetzung in Form einer Paste oder eines Gels vor.

Die erfindungsgemäßen Zusammensetzungen lassen sich beispielsweise durch Fällungsreaktionen aus wässrigen Lösungen wasserlöslicher Calciumsalze und wässrigen Lösungen wasserlöslicher Phosphat- und / oder Fluoridsalze herstellen, wobei die Fällung in Gegenwart von Polyelektrolyten durchgeführt wird und in der Regel durch eine Änderung des pH-Werts, insbesondere durch eine Anhebung oder Erniedrigung auf einen pH-Wert im Bereich zwischen 5 und 10 erfolgt.

Dies kann z. B. in der Weise erfolgen, daß die Polyelektrolyte in reiner, gelöster oder kolloidaler Form der alkalischen wäßrigen Phosphat- und / oder Fluorid-Salzlösung oder der alkalischen Lösung des Calciumsalzes vor der Fällungsreaktion beigefügt werden. Alternativ können die Polyelektrolyte in reiner, gelöster oder kolloidaler Form vorgelegt und anschließend nacheinander in beliebiger Reihenfolge oder gleichzeitig mit der alkalischen Calcium-Salzlösung sowie der alkalischen Phosphat- und / oder Fluorid-Salzlösung versetzt werden. Die Reihenfolge der Zugabe der einzelnen Lösungen ist dabei unkritisch. So können beispielsweise die Polyelektrolyte vorgelegt und die Phosphat- und / oder Fluorid-Salzlösung sowie die Lösung des Calciumsalzes anschließend zugefügt werden. Als Alkalisierungsmittel wird bevorzugt Ammoniak verwendet.

Eine weitere Verfahrensvariante besteht darin, daß man die Fällung aus einer sauren Lösung eines wasserlöslichen Calciumsalzes zusammen mit einer stöchiometrischen Menge eines wasserlöslichen Phosphat- und / oder Fluoridsalzes oder aus einer sauren Lösung von Hydroxylapatit mit einem pH-Wert unterhalb von 5, bevorzugt bei einem pH-Wert unterhalb von 3, durch Anheben des pH-Werts mit wäßrigem Alkali oder Ammoniak in Gegenwart der Polyelektrolyte durchführt.

Eine weitere Verfahrensvariante besteht darin, daß man nanopartikuläre Calciumsalze in reiner oder dispergierter Form oder durch Fällungsreaktionen aus wässrigen Lösungen wasserlöslicher Calciumsalze und wässrigen Lösungen wasserlöslicher Phosphat- und /oder Fluoridsalze hergestellte Dispersionen mit einer Lösung oder Dispersion der Polyelektrolyte versetzt, wobei wiederum die Reihenfolge der Zugabe unkritisch ist.

Die so hergestellte Suspension des nanopartikulären Calciumsalzes wird durch teilweise Entfernung des im Reaktionsmedium befindlichen Wassers, vorzugsweise nach teilweiser oder vollständiger Entfernung der noch vorhandenen gelösten anorganischen Salze, aufkonzentriert und vorzugsweise in eine pasten- oder gelartige Konsistenz überführt. Unter Pasten sind nicht mehr fließfähige Mischungen zu verstehen, was bedeutet, daß das Eigengewicht der Mischung größer ist als seine Fließgrenze.

Die Entfernung der noch vorhandenen gelösten Salze kann beispielsweise durch eine Dialyse oder eine Zentrifugation wie nachstehend beschrieben erfolgen.

Die Überführung der Apatit-Suspension in eine pasten- oder gelartige Konsistenz durch Verminderung des Wassergehalts kann beispielsweise erfolgen durch
- Abdestillation von Wasser, vorzugsweise unter vermindertem Druck. Hierbei bleiben alle nicht flüchtigen Bestandteile des Reaktionsgemischs, u. a. auch die Gegenionen der bei der Fällung benutzten Salze, in der Calciumsalz-Zusammensetzung zurück.
- Zentrifugation, vorzugsweise in einem Drehzahlbereich von 3000 bis 5000 Umdrehungen/Minute. Die Nanopartikel sedimentieren dabei unter Ausbildung eines pastenartigen, wasserhaltigen Niederschlags, der ebenso wie das durch Abdestillation von Wasser erhaltene Produkt vergelen kann. Falls die gelösten Salze weitgehend entfernt werden sollen oder wenn zur Erzielung der gewünschten Konsistenz erforderlich, können Zentrifugation, Abdekantierung der überstehenden Lösung und Ersatz gegen frisches Waschwasser mehrmals wiederholt werden.

Vorzugsweise werden die aus der Fällungsreaktion verbliebenen gelösten Salze so weit entfernt, daß das Dialysewasser bzw. der Überstand aus der Zentrifugation eine Leitfähigkeit von maximal 1000 MikroS/cm aufweist. Der Gesamtsalzgehalt liegt bevorzugt unterhalb von 1 g/l.

Der Wassergehalt in der Paste bzw. dem Gel kann über die Menge des wie vorstehend abgetrennten Wassers eingestellt werden. Wahlweise kann auch zunächst auf einen höheren Feststoffgehalt aufkonzentriert und anschließend durch Wasserzugabe die gewünschte Endkonzentration in der Paste eingestellt werden.

Die Pasten können durch weiteren Wasserentzug und/oder durch mehrstündige bis mehrtägige Lagerung vergelen. Dabei bilden sich durch reversible Agglomeration der Partikel hochporöse temporäre Netzwerke, wodurch die Mischungen selbst eine schnittfeste Konsistenz erhalten können. Diese Netzwerke können unter dem Einfluß von Scherkräften wieder gelöst werden.

Ein weiterer Gegenstand der Erfindung ist somit ein Verfahren zur Herstellung einer Zusammensetzung zur Behandlung von Zahn- und/oder Knochengewebe umfassend einen Polyelektrolyten und ein in Wasser schwerlösliches Calciumsalz, ausgewählt aus Phosphaten, Fluoriden und Fluorophosphaten, die wahlweise zusätzlich Hydroxyl- und/oder Carbonat-Gruppen enthalten können, wobei die Calciumsalze in Form von nanopartikulären, stäbchenförmigen Primärteilchen mit einem mittleren Teilchendurchmesser im Bereich von 5 bis 300 nm vorliegen, dadurch gekennzeichnet, daß es besteht aus den Schritten
(a) Fällung einer wässrigen Lösung eines Calciumsalzes in Gegenwart eines Polyelektrolyten
(b) Verminderung des Wassergehalts bis zum Erreichen einer pasten- oder gelartigen Konsistenz.
   In einer bevorzugten Ausführungsform wird dabei der Schritt (a) derart ausgeführt, daß die Fällung aus einer sauren Lösung eines wasserlöslichen Calciumsalzes und einer stöchiometrischen Menge eines wasserlöslichen Phosphat- und / oder Fluoridsalzes mit einem pH-Wert unterhalb von 3 durch Anheben des pH-Wertes mit wäßrigen Alkalien oder Ammoniak erfolgt.

Schritt (b) wird bevorzugt so ausgeführt, daß die Verminderung des Wassergehalts durch Destillation oder Zentrifugation erfolgt.

Als Polyelektrolyte kommen für das erfindungsgemäße Herstellungsverfahren Polysäuren und Polybasen in Betracht, wobei die Polyelektrolyte Biopolymere oder auch synthetische Polymere sein können. So kann einer oder auch mehrere Polyelektrolyte eingesetzt werden, die ausgewählt sind aus
- Alginsäuren
- Pektinen
- Carrageenan
- Polygalakturonsäuren
- Amino- und Aminosäurederivaten von Alginsäuren, Pektinen, Carrageenan und Polygalakturonsäuren
- Polyaminosäuren, wie z. B. Polyasparaginsäuren
- Polyaspartamiden
- Nucleinsäuren, wie z. B. DNA und RNA
- Ligninsulfonaten
- Carboxymethylcellulosen
- Amino- und/oder Carboxylgruppen-haltigen Cyclodextrin-, Cellulose- oder Dextran-Derivaten
- Polyacrylsäuren
- Polymethacrylsäuren
- Polymaleinaten
- Polyvinylsulfonsäuren
- Polyvinylphosphonsäuren
- Polyethyleniminen
- Polyvinylaminen
   sowie Derivaten der vorstehend genannten Stoffe, insbesondere Amino- und/oder Carboxyl-Derivaten.

Hierbei werden bevorzugt Polyelektrolyte eingesetzt, die zur Salzbildung mit zweiwertigen Kationen geeignete Gruppen tragen. Insbesondere eignen sich Carboxylat-Gruppen tragende Polymere.

Im Sinne der Erfindung im Herstellungsverfahren besonders bevorzugt eingesetzte Polyelektrolyte sind Polyasparaginsäuren, Alginsäuren, Pektine, Desoxyribonukleinsäuren, Ribonukleinsäuren, Polyacrylsäuren und Polymethacrylsäuren. Ganz besonders bevorzugt eingesetzt werden Polyasparaginsäuren mit einem Molekulargewicht im Bereich zwischen ca. 500 und 10000 Dalton, insbesondere 1000 bis 5000 Dalton.

Überraschenderweise wurde gefunden, daß die erfindungsgemäßen Zusammensetzungen bei Wasserentzug zu einem harten, keramikartigen Substrat aushärten, welches sich durch eine hohe Affinität zu Apatitoberflächen, wie sie z. B. an der Oberfläche von Zahn- und Knochengewebe vorliegen, auszeichnet und sich während des Trocknungs- und Aushärtungsvorgangs irreversibel an diese anlagert.

Trägt man beispielsweise die erfindungsgemäßen Zusammensetzungen in dünner Schicht auf eine Oberfläche wie z. B. eine Zahnoberfläche auf, so findet an der Luft eine Trocknung und Aushärtung während eines Zeitraums im Bereich von einigen Sekunden bis einigen Minuten statt. Der zur Aushärtung benötigte Zeitraum hängt im wesentlichen von der Schichtdicke, der Umgebungstemperatur sowie den Bestandteilen der Zusammensetzungen, insbesondere aber ihrem Wassergehalt ab, und kann durch geeignete Wahl dieser Parameter wesentlich beeinflußt werden. Auf diese Weise kann je nach gewünschter Anwendungsweise und -dauer die Verarbeitungszeit der Zusammensetzungen bis zur vollständigen Aushärtung in weiten Grenzen variiert werden.

Ebenfalls Gegenstand der Erfindung ist somit ein Verfahren zur Herstellung eines harten Substrats, welches dadurch gekennzeichnet ist, daß einer wie vorstehend beschriebenen erfindungsgemäßen Zusammensetzung Wasser entzogen wird.

Bei dem Substrat handelt es sich insbesondere um eine Apatit-haltige Beschichtung, welche auf Zahngewebe aufgebracht ist, wobei es sich bei dem Verfahren um kein medizinisches Verfahren handelt.

Die Aushärtung der erfindungsgemäßen Zusammensetzungen bei Wasserentzug findet beispielsweise bei Temperaturen zwischen 0 und 100°C und vorzugsweise zwischen 20 und 40°C statt.

Der Wasserentzug kann auf unterschiedliche Arten erfolgen. In bevorzugter Weise erfolgt er durch isotherme Trocknung der erfindungsgemäßen Zusammensetzung an der Luft. Der Wasserentzug und damit die Aushärtung kann jedoch auch durch Einwirkung von Wärme, wie z. B. durch Einwirkung von warmer Luft, Infrarotstrahlung oder Mikrowellen, und somit eine beschleunigte Verdampfung oder Verdunstung des Wassers, bewirkt oder beschleunigt werden. Weiterhin ist beispielsweise ein Wasserentzug durch Anwendung von reduziertem Druck, gewünschtenfalls in Verbindung mit der Einwirkung von Wärme, möglich.

In einer bevorzugten Ausführungsform erfolgt der Wasserentzug durch Lufttrocknung, besonders bevorzugt bei Temperaturen zwischen 20 und 40°C.

Das erfindungsgemäß durch Wasserentzug gebildete harte Substrat weist eine hohe Affinität zu Zahn- und/oder Knochengewebe auf.

Die erfindungsgemäßen Zusammensetzungen eignen sich demgemäß insbesondere zur Behandlung und/oder Wiederherstellung von Knochen- und Zahngewebe.

Die in den erfindungsgemäßen Zusammensetzungen enthaltenen Polyelektrolyte haften der Oberfläche der feinteiligen Calciumsalze physikalisch an, es kommt jedoch vorzugsweise nicht zu einer chemischen Reaktion mit ihnen. Die einzelnen an der Oberfläche adsorbierten Moleküle sind im wesentlichen frei von intermolekularen Bindungen untereinander. Die Polyelektrolyte haben zum einen die Aufgabe, die nanopartikulären Calciumsalz-Primärteilchen zu stabilisieren und ihre Agglomeration zu verhindern. Weiterhin wird angenommen, daß sie unterschiedliche Primärteilchen brückenartig miteinander verbinden und diese Quervernetzung von Calciumsalz-Partikeln eine wichtige Rolle bei der Aushärtung der Zusammensetzungen spielt. Insbesondere im Falle von Polyasparaginsäure als oberflächenaktiver Substanz wird davon ausgegangen, daß durch ihre strukturelle Ähnlichkeit mit den am natürlichen Biomineralisationsprozeß beteiligten Proteinen die besonders feste Haftung und besonders gute Bioverträglichkeit der erfindungsgemäßen harten Substrate auf Zahn- und Knochenmaterial zustandekommt.

Weiterhin eignen sich die erfindungsgemäßen Zusammensetzungen zur Glättung der Oberfläche von Zahn- und Knochengewebe und Beseitigung von Läsionen und Unebenheiten und damit zur Pflege und zur Reparatur von Defekten insbesondere der Zähne. Die Glättung erfolgt beispielsweise durch Auftragen einer erfindungsgemäßen Zusammensetzung auf den Zahn und anschließendes Polieren. In Experimenten an Rinderzähnen wurde eine etwa zehnfache Verminderung der Mikrorauhigkeit beobachtet. Es wird vermutet, daß dieser glättende Effekt zurückzuführen ist auf abrasive Eigenschaften der in den Zusammensetzungen enthaltenen Calciumsalzpartikel in Kombination mit der Tendenz dieser Nanopartikel, sich auf Apatitoberflächen abzulagern und dabei mikroskopisch kleine Kavitäten und Fissuren aufzufüllen. Durch die Verminderung der Rauhigkeit der Zahnoberfläche wird die Anhaftung von Verunreinigungen vermindert und die Bildung der Plaque gehemmt. Somit wird gleichzeitig eine Prophylaxe der Zähne gegen Bakterienbefall erreicht.

Ein weiterer Gegenstand der Erfindung ist somit die Verwendung der erfindungsgemäßen Zusammensetzungen zur Herstellung eines Arzneimittels zur Glättung der Oberfläche von Zähnen und/oder Knochen.

Die erfindungsgemäßen Zusammensetzungen, insbesondere die von Apatit, Hydroxylapatit und Fluorapatit, fördern und/oder induzieren die Biomineralisation in Zahn- und Knochengewebe. Sie sind daher als solche oder als Komponente in Zubereitungen zur Wiederherstellung oder Neubildung von Zahn- und Knochengewebe wie z. B. Zahnschmelz und Dentin geeignet.

Ein weiterer Gegenstand der Erfindung ist somit die Verwendung der erfindungsgemäßen Zusammensetzungen zur Herstellung eines Arzneimittels zur remineralisierenden Behandlung von Zahn- und/oder Knochengewebe.

Ebenfalls Gegenstand der Erfindung ist die Verwendung der erfindungsgemäßen

Zusammensetzungen zur Herstellung eines Arzneimittels zur Herstellung eines harten Substrats, insbesondere einer Apatit-haltigen Beschichtung auf Zahn- und/oder Knochengewebe.

Die erfindungsgemäßen Zusammensetzungen eignen sich zur Behandlung von Zahn- und Knochendefekten und -frakturen sowie zur Herstellung von Implantaten und zur Förderung des Einwachsens von Implantaten. Weiterhin eignen sie sich als Dentalzemente oder Zusätze zu Dentalzementen, sowie zur Versiegelung oder Reparatur von Fissuren oder sonstigen Defekten von Zahn- und Knochengewebe.

Von besonderem Vorteil ist dabei, daß die erfindungsgemäßen Zusammensetzungen
- gut dosierbar sind
- einfach applizierbar sind, beispielsweise durch Spachtel, Tupfer, Bürsten, Schwämmchen oder Injektionsspritzen
- nicht tropfen, gut verteilbar sind und auch auf unebenen und selbst vertikalen Flächen gut haften sowie
   daß die Aushärtezeit der auf die Zahn- oder Knochenoberfläche aufgetragenen Zusammensetzung der Anwendung entsprechend eingestellt werden kann.

Diese Vorteile wirken sich besonders bei der Anwendung der Zusammensetzungen während einer zahnärztlichen Behandlung am Patienten aus.

In einer Ausführungsform der Erfindung werden die erfindungsgemäßen Zusammensetzungen als remineralisierende und/oder polierende Komponente zur Herstellung von Zubereitungen zur Reinigung und Pflege der Zähne verwendet. Diese Zubereitungen können dabei beispielsweise in Form von Pasten, Gelen, Mundspülungen oder flüssigen Cremes vorliegen. Eine bevorzugte Ausführungsform sind Zahnpasten mit einem Gehalt an Kieselsäure, Poliermitteln, Feuchthaltemitteln, Bindemitteln und Aromen, die 0,5 bis 10 Gew.-% der erfindungsgemäßen Zusammensetzungen enthalten, bezogen auf das Gesamtgewicht der Zubereitung. Die Zubereitungen zur Reinigung und Pflege der Zähne können dabei die üblichen Komponenten und Hilfsmittel solcher Zubereitungen in den dafür üblichen Mengen enthalten.

Die erfindungsgemäßen Zusammensetzungen können je nach beabsichtigter Verwendung weitere Komponenten enthalten, wie z. B.
- Lösungsmittel wie z. B. Aceton oder einwertige Alkohole mit 1 bis 4 Kohlenstoffatomen, z. B. Ethanol oder Isopropanol
- mehrwertige Alkohole mit 2 bis 6 Kohlenstoffatomen und 2 bis 6 Hydroxylgruppen, wie z. B. Glycerin, Ethylenglykol, 1,2-Propylenglycol, Sorbit, Xylit
- Bindemittel und Konsistenzregler, z.B. natürliche und synthetische wasserlösliche Polymere und wasserlösliche Derivate von Naturstoffen, z. B. Celluloseether oder feinteilige Kieselsäuren (Aerogel-Kieselsäuren, pyrogene Kieselsäuren)
- Aromen, z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser und anderer natürlicher und synthetischer Aromen
- Süßstoffe wie z.B. Saccharin-Natrium, Natrium-cyclamat, Aspartame, Acesulfan K, Steviosid, Monellin, Glycyrrhicin, Dulcin, Lactose, Maltose oder Fructose
- Konservierungsmittel und antimikrobielle Stoffe wie z.B. p-Hydroxybenzoesäureester, Natriumsorbat, Triclosan, Hexachlorophen, Phenylsalicylsäureester, Thymol
- Pigmente wie z.B. Titandioxid
- wundheilende und entzündungshemmende Wirkstoffe, z.B. Allantoin, Harnstoff, Azulen, Panthenol, Acetylsalicylsäure-Derivate, Pflanzenextrakte, Vitamine, z.B. Retinol oder Tocopherol.
- die Remineralisation fördernde Wirkstoffe wie Cetylamin-Hydrofluorid.

## Patentansprüche

1. Zusammensetzung zur Behandlung von Zahn- und/oder Knochengewebe umfassend
a) ein in Wasser schwerlösliches Calciumsalz, ausgewählt aus Phosphaten, Fluoriden und Fluorophosphaten, die wahlweise zusätzlich Hydroxyl- und/oder Carbonat-Gruppen enthalten können, wobei die Calciumsalze in Form von nanopartikulären, stäbchenförmigen Primärteilchen mit einem mittleren Teilchendurchmesser im Bereich von 5 bis 300 nm vorliegen, und
b) einen Polyelektrolyten ausgewählt aus Alginsäuren, Pektinen, Carrageenan, Polygalakturonsäuren, Amino- und Aminosäurederivaten von Alginsäuren, Pektinen, Carrageenan und Polygalakturonsäuren, Polyaspartamiden, Nucleinsäuren, Ligninsulfonaten, Amino- und/oder Carboxylgruppen-haltigen Cyclodextrin- oder Dextran-Derivaten, Polymethacrylsäuren, Polymaleinaten, Polyvinylsulfonsäuren, Polyvinylphosphonsäuren, Polyethyleniminen, Polyvinylaminen,
erhältlich durch ein Verfahren, bestehend aus den Schritten
- Fällung einer wässrigen Lösung eines Calciumsalzes in Gegenwart eines Polyelektrolyten
- Verminderung des Wassergehalts bis zum Erreichen einer pasten- oder gelartigen Konsistenz.

2. Zusammensetzung nach Anspruch 1, **dadurch gekennzeichnet, daß** das Calciumsalz ausgewählt ist aus Apatit, Hydroxylapatit und Fluorapatit.

3. Zusammensetzung nach einem der Ansprüche 1 oder 2, **dadurch gekennzeichnet, daß** der Polyelektrolyt ausgewählt ist aus der Gruppe, die gebildet wird von Alginsäuren, Pektinen, Desoxyribonukleinsäuren, Ribonukleinsäuren und Polymethacrylsäuren.

4. Zusammensetzung nach einem der Ansprüche 1 bis 3, **dadurch gekennzeichnet, daß** der Anteil des Calciumsalzes in der Zusammensetzung zwischen 1 und 40 Gew.-%, vorzugsweise zwischen 5 und 25 Gew.-%, bezogen auf das Gesamtgewicht der Zusammensetzung beträgt.

5. Zusammensetzung nach einem der Ansprüche 1 bis 4, **dadurch gekennzeichnet, daß** der Anteil des Polyelektrolyts in der Zusammensetzung zwischen 0,1 und 40 Gew.-%, vorzugsweise zwischen 5 und 20 Gew.-%, bezogen auf das Gesamtgewicht der Zusammensetzung beträgt.

6. Zusammensetzung nach einem der Ansprüche 1 bis 5, **dadurch gekennzeichnet, daß** der Anteil des Polyelektrolyts in der Zusammensetzung zwischen 2 und 50 Gew.-% und bevorzugt zwischen 5 und 10 Gew.-% bezogen auf das Gesamtgewicht aus Calciumsalz und Polyelektrolyt beträgt.

7. Zusammensetzung nach einem der Ansprüche 1 bis 6, **dadurch gekennzeichnet, daß** sie zwischen 10 und 95 Gew.-%, vorzugsweise zwischen 50 und 80 Gew.-%, Wasser enthält, bezogen auf das Gesamtgewicht der Zusammensetzung.

8. Zusammensetzung nach einem der Ansprüche 1 bis 7, **dadurch gekennzeichnet, daß** sie in Form einer Paste oder eines Gels vorliegt.

9. Verfahren zur Herstellung einer Zusammensetzung zur Behandlung von Zahn- und/oder Knochengewebe umfassend einen Polyelektrolyten und ein in Wasser schwerlösliches Calciumsalz, ausgewählt aus Phosphaten, Fluoriden und Fluorophosphaten, die wahlweise zusätzlich Hydroxyl- und/oder Carbonat-Gruppen enthalten können, wobei die Calciumsalze in Form von nanopartikulären, stäbchenförmigen Primärteilchen mit einem mittleren Teilchendurchmesser im Bereich von 5 bis 300 nm vorliegen, **dadurch gekennzeichnet, daß** es besteht aus den Schritten
(a) Fällung einer wässrigen Lösung eines Calciumsalzes in Gegenwart eines Polyelektrolyten
(b) Verminderung des Wassergehalts bis zum Erreichen einer pasten- oder gelartigen Konsistenz.

10. Verfahren nach Anspruch 9, **dadurch gekennzeichnet, daß** die Fällung aus einer sauren Lösung eines wasserlöslichen Calciumsalzes und einer stöchiometrischen Menge eines wasserlöslichen Phosphat- und / oder Fluoridsalzes mit einem pH-Wert unterhalb von 3 durch Anheben des pH-Wertes mit wässrigen Alkalien oder Ammoniak erfolgt.

11. Verfahren nach einem der Ansprüche 9 oder 10, **dadurch gekennzeichnet, daß** die Verminderung des Wassergehalts durch Destillation oder Zentrifugation erfolgt.

12. Verfahren zur Herstellung eines harten Substrats, **dadurch gekennzeichnet, daß** einer Zusammensetzung nach einem der Ansprüche 1 bis 8 Wasser entzogen wird.

13. Verfahren nach Anspruch 12, **dadurch gekennzeichnet, daß** es sich bei dem Substrat um eine Apatit-haltige Beschichtung handelt, welche auf Zahngewebe aufgebracht ist, wobei es sich bei dem Verfahren um kein medizinisches Verfahren handelt.

14. Verfahren nach Anspruch 12 oder 13, **dadurch gekennzeichnet, daß** der Wasserentzug in einem Temperaturbereich zwischen 0 bis 100°C und vorzugsweise zwischen 20 und 40°C erfolgt.

15. Verfahren nach einem der Ansprüche 12 bis 14, **dadurch gekennzeichnet, daß** der Wasserentzug durch Lufttrocknung erfolgt.

16. Verfahren nach einem der Ansprüche 12 bis 15, **dadurch gekennzeichnet, daß** das durch Wasserentzug aus der Zusammensetzung gebildete harte Substrat eine hohe Affinität zu Zahn- und/oder Knochengewebe aufweist.

17. Verwendung einer Zusammensetzung nach einem der Ansprüche 1 bis 8 zur Herstellung eines Arzneimittels zur Glättung der Oberfläche von Zähnen und/oder Knochen.

18. Verwendung einer Zusammensetzung nach einem der Ansprüche 1 bis 8 zur Herstellung eines Arzneimittels zur remineralisierenden Behandlung von Zahn- und/oder Knochengewebe.

19. Verwendung einer Zusammensetzung nach einem der Ansprüche 1 bis 8 zur Herstellung eines Arzneimittels zur Herstellung eines harten Substrats, insbesondere einer Apatit-haltigen Beschichtung auf Zahn- und/oder Knochengewebe.

## Claims

1. Composition for treating tooth and/or bone tissue, comprising
a) a calcium salt of low solubility in water, selected from phosphates, fluorides and fluorophosphates, which optionally may additionally contain hydroxyl and/or carbonate groups, where the calcium salts are in the form of nanoparticulate, rod-like primary particles with an average particle diameter in the range from 5 to 300 nm, and
b) a polyelectrolyte selected from alginic acids, pectins, carrageenan, polygalacturonic acids, amino and amino acid derivatives of alginic acids, pectins, carrageenan and polygalacturonic acids, polyaspartamides, nucleic acids, ligninsulfonates, amino and/or carboxyl group-containing cyclodextrin or dextran derivatives, polymethacrylic acids, polymaleates, polyvinylsulfonic acids, polyvinylphosphonic acids, polyethyleneimines, polyvinylamines, obtainable by a process consisting of the steps of
- precipitation of an aqueous solution of a calcium salt in the presence of a polyelectrolyte
- reduction of the water content until a pasty or gelatinous consistency is reached.

2. Composition according to Claim 1, **characterized in that** the calcium salt is selected from apatite, hydroxyapatite and fluoroapatite.

3. Composition according to either of Claims 1 or 2, **characterized in that** the polyelectrolyte is selected from the group formed by alginic acids, pectins, deoxyribonucleic acids, ribonucleic acids and polymethacrylic acids.

4. Composition according to any of Claims 1 to 3, **characterized in that** the proportion of the calcium salt in the composition is between 1 and 40% by weight, preferably between 5 and 25% by weight, based on the total weight of the composition.

5. Composition according to any of Claims 1 to 4, **characterized in that** the proportion of the polyelectrolyte in the composition is between 0.1 and 40% by weight, preferably between 5 and 20% by weight, based on the total weight of the composition.

6. Composition according to any of Claims 1 to 5, **characterized in that** the proportion of the polyelectrolyte in the composition is between 2 and 50% by weight and preferably between 5 and 10% by weight, based on the total weight of calcium salt and polyelectrolyte.

7. Composition according to any of Claims 1 to 6, **characterized in that** it contains between 10 and 95% by weight, preferably between 50 and 80% by weight, of water, based on the total weight of the composition.

8. Composition according to any of Claims 1 to 7, **characterized in that** it is in the form of a paste or a gel.

9. Process for producing a composition for treating tooth and/or bone tissue, comprising a polyelectrolyte and a calcium salt of low solubility in water, selected from phosphates, fluorides and fluorophosphates, which optionally may additionally contain hydroxyl and/or carbonate groups, where the calcium salts are in the form of nanoparticulate, rod-like primary particles with an average particle diameter in the range from 5 to 300 nm, **characterized in that** it consists of the steps of
(a) precipitation of an aqueous solution of a calcium salt in the presence of a polyelectrolyte
(b) reduction of the water content until a pasty or gelatinous consistency is reached.

10. Process according to Claim 9, **characterized in that** the precipitation takes place from an acidic solution of a water-soluble calcium salt and of a stoichiometric amount of a water-soluble phosphate and/or fluoride salt with a pH below 3 by raising the pH with aqueous alkalis or ammonia.

11. Process according to either of Claims 9 or 10, **characterized in that** the water content is reduced by distillation or centrifugation.

12. Process for producing a hard substrate, **characterized in that** water is removed from a composition according to any of Claims 1 to 8.

13. Process according to Claim 12, **characterized in that** the substrate is an apatite-containing coating which is applied to tooth tissue, where the process is not a medical process.

14. Process according to Claim 12 or 13, **characterized in that** the removal of water takes place in a temperature range from 0 to 100°C and preferably between 20 and 40°C.

15. Process according to any of Claims 12 to 14, **characterized in that** the removal of water takes place by drying in air.

16. Process according to any of Claims 12 to 15, **characterized in that** the hard substrate formed by removal of water from the composition has a high affinity for tooth and/or bone tissue.

17. Use of a composition according to any of Claims 1 to 8 for producing a medicament for smoothing the surface of teeth and/or bones.

18. Use of a composition according to any of Claims 1 to 8 for producing a medicament for the remineralizing treatment of tooth and/or bone tissue.

19. Use of a composition according to any of Claims 1 to 8 for producing a medicament for producing a hard substrate, in particular an apatite-containing coating on tooth and/or bone tissue.

## Revendications

1. Composition pour traiter les tissus dentaires et/ou osseux comprenant
a) un sel de calcium peu soluble dans l'eau, choisi parmi les phosphates, les fluorures et les fluorophosphates, qui peuvent contenir au choix en sus des groupes hydroxyle et/ou carbonate, les sels de calcium existant sous forme de particules primaires nanoparticulaires, en forme de bâtonnets, ayant un diamètre moyen de particule dans la plage de 5 à 300 nm, et
b) un polyélectrolyte choisi parmi les acides alginiques, les pectines, les carraghénanes, les poly(acides galacto-uronique)s, les dérivés amino et amino-acide des acides alginiques, les pectines, les carraghénanes et les poly(acide galacto-uronique)s, les polyaspartamides, des acides nucléiques, les sulfonates de lignine, les dérivés de cyclodextrine ou de dextrane contenant des groupes amino et/ou carboxyle, les poly(acide méthacrylique)s, les polymaléates, les poly(acide vinylsulfonique)s, les poly(acide vinylphosphonique)s, les pclyéthylène-imines, les polyvinylamines,
pouvant être obtenus à l'aide d'un procédé se composant des étapes de
- précipitation d'une solution aqueuse d'un sel de calcium en présence d'un polyélectrolyte
- réduction de la teneur en eau jusqu'à l'obtention d'une consistance pâteuse ou de type gel.

2. Composition selon la revendication 1, **caractérisée en ce que** le sel de calcium est choisi parmi l'apatite, l'hydroxylapatite, et la fluoroapatite.

3. Composition selon l'une quelconque des revendications 1 ou 2,
**caractérisée en ce que** le polyélectrolyte est choisi dans le groupe qui est formé par les acides alginiques, les pectines, les acides désoxyribonucléiques, les acides ribonucléiques, et les poly(acide méthacrylique)s.

4. Composition selon l'une quelconque des revendications 1 à 3, **caractérisée en ce que** la proportion du sel de calcium dans la composition est comprise entre 1 et 40% en poids, de préférence entre 5 et 25% en poids, par rapport au poids total de la composition.

5. Composition selon l'une quelconque des revendications 1 à 4, **caractérisée en ce que** la proportion du polyélectrolyte dans la composition est comprise entre 0,1 et 40% en poids, de préférence entre 5 et 20% en poids, par rapport au poids total de la composition.

6. Composition selon l'une quelconque des revendications 1 à 5, **caractérisée en ce que** la proportion du polyélectrolyte dans la composition est comprise entre 2 et 50% en poids, de préférence entre 5 et 10% en poids, par rapport au poids total du sel de calcium et du polyélectrolyte.

7. Composition selon l'une quelconque des revendications 1 à 6, **caractérisée en ce qu'**elle contient entre 10 et 95% en poids, de préférence entre 50 et 80% en poids d'eau, par rapport au poids total de la composition.

8. Composition selon l'une quelconque des revendications 1 à 7, **caractérisée en ce qu'**elle existe sous forme d'une pâte ou d'un gel.

9. Procédé pour préparer une composition destinée à traiter les tissus dentaires et/ou osseux comprenant un polyélectrolyte et un sel de calcium peu soluble dans l'eau, choisi parmi les phosphates, les fluorures et les fluorophosphates, qui peuvent contenir au choix en sus des groupes hydroxyle et/ou carbonate, les sels de calcium existant sous forme de particules primaires nanoparticulaires, en forme de bâtonnets, ayant un diamètre moyen de particule dans la plage de 5 à 300 nm, **caractérisé en ce qu'**il se compose des étapes de
a) précipitation d'une solution aqueuse d'un sel de calcium en présence d'un polyélectrolyte
b) réduction de la teneur en eau jusqu'à l'obtention d'une consistance pâteuse ou de type gel.

10. Procédé selon la revendication 9, **caractérisé en ce que** la précipitation dans une solution acide d'un sel de calcium hydrosoluble et d'une quantité stoechiométrique d'un sel phosphate et/ou fluorure hydrosoluble ayant une valeur de pH inférieure à 3, se réalise en élevant la valeur de pH avec des alcalis aqueux ou de l'ammoniaque.

11. Procédé selon l'une quelconque des revendications 9 ou 10, **caractérisé en ce que** la réduction de la teneur en eau se réalise par distillation ou centrifugation.

12. Procédé pour préparer un substrat dur, **caractérisé en ce que** de l'eau est retirée d'une composition selon l'une quelconque des revendications 1 à 8.

13. Procédé selon la revendication 12, **caractérisé en ce que**, quant au substrat, il s'agit d'un revêtement contenant de l'apatite, qui est appliqué sur les tissus dentaire, où quant au procédé, il s'agit d'un procédé médicinal.

14. Procédé selon la revendication 12 ou 13, **caractérisé en ce que** le retrait d'eau se réalise dans une plage de températures comprise entre 0 à 100°C et de préférence entre 20 et 40°C.

15. Procédé selon l'une quelconque des revendications 12 à 14, **caractérisé en ce que** le retrait d'eau se réalise par séchage à l'air.

16. Procédé selon l'une quelconque des revendications 12 à 14, **caractérisé en ce que** le substrat dur formé par le retrait d'eau de la composition présente une affinité élevée pour les tissus dentaires et/ou osseux.

17. Utilisation d'une composition selon l'une quelconque des revendications 1 à 8, pour préparer un médicament pour le lissage de la surface de dents et/ou d'os.

18. Utilisation d'une composition selon l'une quelconque des revendications 1 à 8 pour préparer un médicament pour le traitement reminéralisant de tissus dentaires et/ou osseux.

19. Utilisation d'un composition selon l'une quelconque des revendications 1 à 8 pour préparer un médicament afin de préparer un substrat dur, en particulier un revêtement contenant de l'apatite sur des tissus dentaires et/ou osseux.
